# EUROPEAN PATENT APPLICATION

(11) **EP 4 336 169 A1**
(43) Date of publication of application: **13.03.2024**
(21) Application number: 22798953.0
(22) Date of filing: 02.05.2022
(51) Int. Cl.: G01N 21/01, G01N 21/27, G01N 21/64, G01N 27/06

(54) **METHOD FOR DETECTING SUBSTANCE TO BE DETECTED, DETECTION KIT AND DETECTION SYSTEM, AND METHOD FOR MANUFACTURING DETECTION KIT**

(30) Priority: 07.05.2021 JP 2021079295
(71) Applicant: University Public Corporation Osaka, Osaka City 545-0051 (JP)
(72) Inventor: TOKONAMI, Shiho, Sakai-shi, Osaka 599-8531 (JP); IIDA, Takuya, Sakai-shi, Osaka 599-8531 (JP)
(74) Representative: Bals & Vogel Patentanwälte PartGmbB
(86) International application number: PCT/JP2022/019532
(87) International publication number: WO 2022/234849

(57) **Abstract**

A detection kit (10) includes a photothermal conversion region (a honeycomb polymer membrane (12) and a thin film (13)) that absorbs light and converts the light into heat. A plurality of pores (14) are disposed in the photothermal conversion region. A detection method includes first to third steps. The first step is introducing a plurality of antibody-modified beads (23) into the plurality of pores (14), each of the plurality of antibody-modified beads (23) having a surface modified by an antibody (22) capable of being specifically bound to an analyte (bacteria). The second step is heating a sample to generate convection in the sample by irradiating the photothermal conversion region with light having a wavelength within an absorption wavelength range of the photothermal conversion region. The third step is detecting bacteria by monitoring the detection kit (10) after the irradiation with the light.

## Description

### TECHNICAL FIELD

The present disclosure relates to a method for detecting an analyte, a detection kit and a detection system, and a method for manufacturing the detection kit, and more specifically relates to a technique for detecting an analyte that may be contained in a liquid sample.

### BACKGROUND ART

Japanese Patent Laying-Open No. 2017-202446 (PTL 1) discloses a collecting device for microscopic objects, the device collecting a plurality of microscopic objects dispersed in a liquid. The correcting device includes a light source for emitting light and a holding member configured to hold a liquid. In the holding member, an inner wall part for specifying a space for trapping a plurality of microscopic objects dispersed in the liquid is formed, and a photothermal conversion region containing a material that converts light from a light source into heat is formed. The photothermal conversion region generates convection in the liquid by converting light from the light source into heat and applying the heat to the liquid.

### CITATION LIST

### PATENT LITERATURES

PTL 1: Japanese Patent Laying-Open No. 2017-202446
PTL 2: WO 2018/207937
PTL 3: Japanese Patent No. 6375578

### SUMMARY OF INVENTION

### TECHNICAL PROBLEM

A technique for selectively and quickly detecting an analyte that may be contained in a liquid has been constantly in demand.

The present disclosure has been devised to solve the above problem. An object of the present disclosure is to selectively and quickly detect an analyte that may be contained in a liquid sample.

### SOLUTION TO PROBLEM

According to an aspect of the present disclosure, a method for detecting an analyte detects an analyte that may be contained in a liquid sample by using a detection kit. The detection kit includes a photothermal conversion region that absorbs light and converts the light into heat. A plurality of pores are disposed in the photothermal conversion region. The detection method includes first to third steps. The first step is introducing a plurality of microscopic objects into the plurality of pores, each of the plurality of microscopic objects having a surface modified by a host substance capable of being specifically (selectively) bound to the analyte. The second step is heating the liquid sample to generate thermal convection in the liquid sample by irradiating the photothermal conversion region with light having a wavelength within an absorption wavelength range of the photothermal conversion region. The third step is detecting the analyte, by monitoring the detection kit after the irradiation with the light.

According to another aspect of the present disclosure, a detection kit for an analyte is used for detecting the analyte that may be contained in a liquid sample. The detection kit includes a photothermal conversion region that absorbs light and converts the light into heat. A plurality of pores are disposed in the photothermal conversion region. The detection kit further includes a plurality of microscopic objects disposed in the plurality of pores, each of the plurality of microscopic objects having a surface modified by a host substance capable of being specifically bound to the analyte.

According to still another aspect of the present disclosure, a detection system of an analyte includes the detection kit, a light source that emits light having a wavelength within the absorption wavelength range of the photothermal conversion region, and a detector that detects the analyte, by monitoring the detection kit after irradiation with the light from the light source.

According to still another aspect of the present disclosure, a detection kit is used for detecting an analyte that may be contained in a liquid sample. A method for manufacturing a detection kit includes: preparing the detection kit including a photothermal conversion region in which a plurality of pores are disposed; and introducing a plurality of microscopic objects into the plurality of pores, each of the plurality of microscopic objects having a surface modified by a host substance capable of being specifically bound to the analyte.

### ADVANTAGEOUS EFFECT OF INVENTION

According to the present disclosure, an analyte that may be contained in a liquid sample can be selectively and quickly detected.

### BRIEF DESCRIPTION OF DRAWINGS

Fig. 1 is a diagram of the overall configuration of a detection system of bacteria according to Embodiment 1.
Fig. 2 is a conceptual diagram for illustrating the configuration of a detection kit.
Fig. 3 is a cross-sectional view of the detection kit taken along line III-III of Fig. 2.
Fig. 4 is a diagram illustrating a top SEM image of a honeycomb polymer membrane produced according to the present embodiment.
Fig. 5 is a diagram illustrating a perspective SEM image of the honeycomb polymer membrane manufactured according to the present embodiment.
Fig. 6 is a diagram illustrating a microscopic object according to the present embodiment.
Fig. 7 is a conceptual diagram illustrating a state of antibody-modified beads introduced into a plurality of pores disposed on the honeycomb polymer membrane.
Fig. 8 is a diagram illustrating an optical transmission image of the top surface of the detection kit where the antibody-modified beads are introduced.
Fig. 9 is an enlarged view of the optical transmission image shown in Fig. 8.
Fig. 10 is a conceptual diagram for illustrating a state of bacteria accumulated by using the detection kit.
Fig. 11 is a diagram for explaining an accumulation pattern of bacteria in a comparative example.
Fig. 12 is a diagram for explaining an accumulation pattern of bacteria in the present embodiment.
Fig. 13 is a flowchart indicating the steps of a method for detecting bacteria according to Embodiment 1.
Fig. 14 is a diagram illustrating the images of the detection kit in the presence/absence of irradiation with a laser beam at a wavelength of 1064 nm and the extraction result of an area where bacteria are accumulated.
Fig. 15 is a diagram illustrating the accumulation results of bacteria in samples with different bacterial concentrations.
Fig. 16 is a diagram illustrating the correlation between a bacterial concentration and a fluorescence area (the accumulation area of bacteria), the correlation being determined from the images shown in Fig. 15.
Fig. 17 is a diagram illustrating the accumulation results of various kinds of bacteria.
Fig. 18 is a diagram illustrating the calculation result of a fluorescence area for each kind of bacteria, the calculation result being determined from the images shown in Fig. 17.
Fig. 19 is a diagram illustrating the accumulation results of bacteria in mixed bacteria samples containing two kinds of bacteria.
Fig. 20 is a diagram illustrating the calculation result of a fluorescence area (the number of measurements n = 3), the calculation result being determined from the images shown in Fig. 19.
Fig. 21 is a diagram illustrating the accumulation results of bacteria in mixed bacteria samples containing four kinds of bacteria.
Fig. 22 is a diagram illustrating the calculation result of a fluorescence area (the number of measurements n = 3), the calculation result being determined from the images shown in Fig. 21.
Fig. 23 is a diagram illustrating the accumulation results of bacteria in impurity samples.
Fig. 24 is a diagram illustrating the correlation between a bacterial concentration and a fluorescence area, the correlation being determined from the images shown in Fig. 23.
Fig. 25 is a diagram illustrating the accumulation results of various kinds of bacteria in impurity samples.
Fig. 26 is a diagram illustrating the calculation result of a fluorescence area for each kind of bacteria (the number of measurements n = 3), the calculation result being determined from the images shown in Fig. 25.
Fig. 27 is a diagram of the overall configuration of a detection system of bacteria according to Embodiment 2.
Fig. 28 is a flowchart indicating the steps of a method for detecting bacteria according to Embodiment 2.
Fig. 29 is a diagram of the overall configuration of a detection system of bacteria according to Embodiment 3.
Fig. 30 is a diagram for explaining the detail of the configuration of a detection kit according to Embodiment 3.
Fig. 31 is a flowchart indicating the steps of a method for detecting bacteria according to Embodiment 3.

### DESCRIPTION OF EMBODIMENTS

### <Definition of Terms>

In the present disclosure and embodiments, "nanometer order" includes a range from 1 nm to 1000 nm (= 1µm). "Micrometer order" includes a range from 1 µm to 1000 µm (= 1 mm). Thus, "a range from the nanometer order to the micrometer order" includes a range from 1 nm to 1000 µm. "A range from the nanometer order to the micrometer order" typically indicates a range from several nm to several hundred µm, preferably indicates a range from 100 nm to 100 µm, and more preferably indicates a range from several hundred nm to several tens of µm.

In the present disclosure and embodiments, "sample" means a substance containing an analyte or a substance that may contain an analyte. The sample can be, for example, a biological sample from animals (such as a human, a cow, a horse, a pig, a goat, a chicken, a rat, and a mouse). The biological sample may contain, for example, blood, tissues, cells, secretory fluid, and body fluid. "Sample" may contain a dilution thereof. The sample may be a food-derived substance.

In the present disclosure and embodiments, "analyte" means a substance that has a size ranging from the nanometer order to the micrometer order and is detected by using a detection kit. The shape of the analyte is not particularly limited and may be, for example, a sphere, an oval sphere, or a rod (pole). If the analyte is shaped like an oval sphere, the oval sphere may have a length in a range from the nanometer order to the micrometer order in at least one of the major axis direction and the minor axis direction of the oval sphere. If the analyte is shaped like a rod, at least one of the width and the length of the rod may be set in a range from the nanometer order to the micrometer order.

Examples of the analyte include cells, microorganisms (such as a germ and a fungus), low molecules (molecules with a molecular weight of about several hundred), middle molecules (molecules with a molecular weight of about 500 to 2000), biopolymers (such as protein, nucleic acid, lipid, and polysaccharide), antibodies, antigens (such as an allergen), and viruses. However, the analyte is not limited to a tissue-derived material (biological substance). The analyte may be, for example, metallic nanoparticles, metallic nanoparticle assemblies, metallic nanoparticle accumulated structures, semiconductor nanoparticles, organic nanoparticles, resin beads, and microparticles. "Metallic nanoparticles" are metallic particles in the size of nanometer order. "Metallic nanoparticle assemblies" are assemblies formed by the aggregation of a plurality of metallic nanoparticles. "Metallic nanoparticle accumulated structures" are structures in which, for example, a plurality of metallic nanoparticles are fixed on the surfaces of beads with an interaction portion interposed therebetween, with gaps between each other and spaced at intervals equal to or smaller than the diameter of the metallic nanoparticle. "Semiconductor nanoparticles" are semiconductor particles in the size of nanometer order. "Organic nanoparticles" are particles containing an organic compound in the size of nanometer order. "Resin beads" are resin particles sized from the nanometer order to the micrometer order. "microparticles" are particles in the size of micrometer order and include, for example, metallic microparticles, semiconductor microparticles, and resin microbeads. Microparticles may also include toxic microparticles such as PM2.5 and microplastics.

In the present disclosure and embodiments, "host substance" means a substance capable of specifically binding the analyte. Examples of combinations of a host substance capable of specifically binding an analyte and the analyte include: an antigen and an antibody, a sugar chain and a protein, a lipid and a protein, a low-molecular compound (ligand) and a protein, a protein and a protein, a single-stranded DNA and a single-stranded DNA, and a protein and a nucleic acid molecule (aptamer). If one of the substances having specific affinity is an analyte, the other can be used as a host substance. Specifically, if an antigen is an analyte, an antibody can be used as a host substance. Conversely, if an antibody is an analyte, an antigen can be used as a host substance. In DNA hybridization, an analyte is a target DNA and a host substance is a probe DNA. Antigens may include, for example, an allergen, microorganisms (such as a germ and a fungus), and viruses. Moreover, the kind of detectable allergen or virus can be changed by changing the kind of antibody. Thus, the kind of detectable allergen or virus according to the present disclosure is not particularly limited. If an analyte is a heavy metal, a substance capable of trapping a heavy metal ion can be used as a host substance.

In the present disclosure and embodiments, "microscopic object" means an object in sizes ranging from the nanometer order to the micrometer order. Like an analyte, the shape of the microscopic object is not particularly limited and may be shaped like, for example, a sphere, an oval sphere, or a rod. If the microscopic object is shaped like an oval sphere, the oval sphere may have a length in a range from the nanometer order to the micrometer order in at least one of the major axis direction and the minor axis direction of the oval sphere. If the microscopic object is shaped like a rod, at least one of the width and the length of the rod may be set in a range from the nanometer order to the micrometer order.

In the present disclosure and embodiments, "absorb light" means a property that the intensity of light absorbed by a substance is larger than 0. The wavelength range of light may be any one of an ultraviolet range, a visible range, and a near-infrared range, a range extending over two of the three ranges, or a range extending over all the three ranges. Light absorption can be defined by, for example, the range of the absorptivity of light. In this case, the lower limit of the range of absorptivity is not particularly limited as long as the lower limit is higher than 0. The upper limit of the range of absorptivity is 100%.

In the present disclosure and embodiments, "honeycomb pattern" is a shape having a plurality of regular hexagons arranged in a hexagonal lattice (like a honeycomb) in the two-dimensional direction. A pore is formed for each of the regular hexagons. A structure in which a plurality of pores are arranged in a honeycomb pattern will be referred to as "honeycomb structure." The pores are holes having openings in sizes ranging from the nanometer order to the micrometer order. The pores may be penetrating pores or non-penetrating pores. The shape of the pore is not particularly limited and may include any shapes such as a cylinder, a prism, and a sphere other than a perfect sphere (e.g., a hemisphere or a semi-elliptical sphere).

In the present disclosure and embodiments, "microbubble" means a bubble on the micrometer order.

In the present disclosure and embodiments, "visible range" means a wavelength range of 360 nm to 760 nm. A near-infrared range means a wavelength range of 760 nm to 2 µm.

The embodiments according to the present disclosure will be specifically described below with reference to the accompanying drawings. Hereinafter, the same parts or equivalent parts in the drawings are denoted by the same reference numerals and a repetition of a description thereof is omitted. The x direction and the y direction indicate horizontal directions. The x direction and the y direction are orthogonal to each other. The z direction indicates the vertical direction. Gravity is directed downward in the z direction. Directed upward in the z direction may be abbreviated as "upward" and directed downward in the z direction may be abbreviated as "downward. "

### [Embodiment 1]

The present embodiment will describe an example in which an analyte is bacteria. Bacteria to be detected will be also referred to as "target bacteria."

### <Overall Configuration of Detection System>

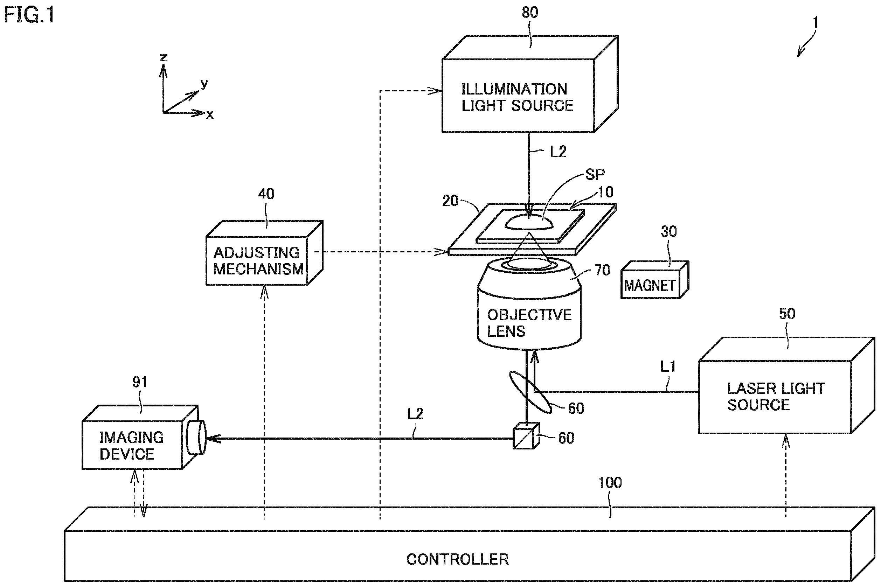

Fig. 1 is a diagram of the overall configuration of a detection system 1 of bacteria according to Embodiment 1. Detection system 1 includes a detection kit 10, an xyz-axis stage 20, a magnet 30, an adjusting mechanism 40, a laser light source 50, optical components 60, an objective lens 70, an illumination light source 80, an imaging device 91, and a controller 100.

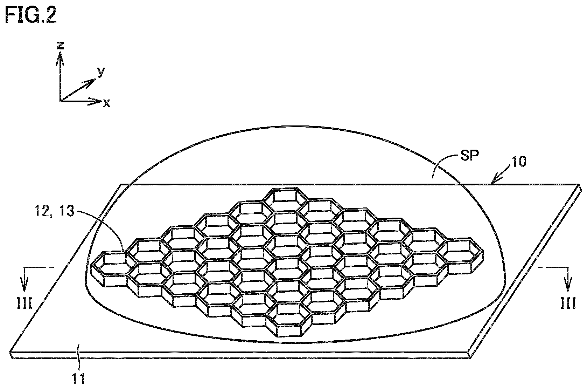

Detection kit 10 holds a dropped sample (denoted as SP). In the present embodiment, the sample is a liquid sample that may contain target bacteria. Detection kit 10 is placed on xyz-axis stage 20. The specific configuration of detection kit 10 will be described referring to Figs. 2 to 5.

Xyz-axis stage 20 is configured to move by adjusting mechanism 40 in x-direction, y-direction, and z-direction.

Magnet 30 is disposed below detection kit 10 and is configured to apply an external magnetic field to detection kit 10. Magnet 30 may be a permanent magnet (e.g., a ferrite magnet or a neodymium magnet) or an electromagnet. If magnet 30 is an electromagnet, energization/non-energization of magnet 30 may be controlled by controller 100. Magnet 30 is used when introducing microscopic objects to detection kit 10 (described later). Therefore, magnet 30 is disposed below detection kit 10 at the time of the introduction of microscopic objects; and is configured to move to a location other than the location below detection kit 10 during light concentration (described later) using laser light source 50.

Adjusting mechanism 40 adjusts the position of xyz-axis stage 20 in x-direction, y-direction, and z-direction according to a command from controller 100. In the present embodiment, the position of objective lens 70 is fixed, so that the relative positional relationship between detection kit 10 and objective lens 70 is adjusted by adjusting the position of xyz-axis stage 20. As adjusting mechanism 40, for example, drive mechanisms such as a servo motor and a focusing handle provided for a microscope may be used. The specific configuration of adjusting mechanism 40 is not particularly limited. Adjusting mechanism 40 may be configured to adjust the position of objective lens 70 with respect to fixed detection kit 10.

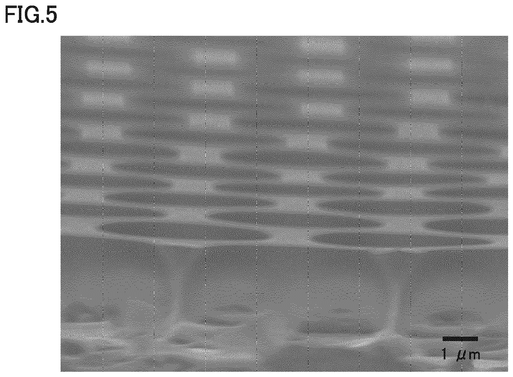

Laser light source 50 emits a laser beam (denoted as L1) of a continuous wave (CW) according to a command from controller 100. The wavelength of the laser beam is a wavelength within the absorption wavelength range of a thin film 13 (see Figs. 2 and 5), for example, the wavelength is a wavelength of a near-infrared range (e.g., 800 nm and 1064). The wavelength of the laser beam may be a wavelength within a visible range.

Optical components 60 include, for example, a mirror, a dichroic mirror, and a prism. The optical system of detection system 1 is adjusted such that a laser beam from laser light source 50 is guided to objective lens 70 by optical components 60.

Objective lens 70 condenses a laser beam from laser light source 50. Light condensed through objective lens 70 is emitted to detection kit 10. In this case, "emitted" includes a case where a laser beam passes through detection kit 10. Specifically, it is not limited to a case where the beam waist of light condensed through objective lens 70 is located in detection kit 10. Optical components 60 and objective lens 70 can be assembled into, for example, an inverted microscope body or an upright microscope body. In this example, objective lens 70 is assembled into an inverted microscope body with a magnification of 40 times (dry).

Illumination light source 80 emits white light (denoted as L2) for illuminating a sample on detection kit 10, according to a command from controller 100. As an example, a halogen lamp can be used as illumination light source 80. Objective lens 70 is used also for bringing in white light emitted to detection kit 10 from illumination light source 80. White light brought in by objective lens 70 is guided to imaging device 91 by optical components 60.

Imaging device 91 captures an image of the sample on detection kit 10 irradiated with white light and outputs the captured image to controller 100 according to a command from controller 100. The image captured by imaging device 91 may be a still image or a moving image. As imaging device 91, a camera including a CCD (Charge Coupled Device) image sensor or a CMOS (Complementary Metal Oxide Semiconductor) image sensor can be used. Imaging device 91 is an example of "light receiver" and "detector" according to the present disclosure.

Controller 100 includes a processor, e.g., a CPU (Central Processing Unit), a memory, e.g., a ROM (Read Only Memory) and a RAM (Random Access Memory), and an input/output port for obtaining various signals. The processor, the memory, and the input/output port are not illustrated. Controller 100 controls the devices (adjusting mechanism 40, laser light source 50, illumination light source 80, and imaging device 91) in detection system 100. Moreover, controller 100 detects target bacteria in the sample by performing predetermined image processing on an image captured by imaging device 91.

The optical system of detection system 1 is not limited to the configuration shown in Fig. 1 as long as a laser beam from laser light source 50 can be emitted to detection kit 10 and white light from detection kit 10 can be brought into imaging device 91. The optical system of detection system 1 may include, for example, other optical components (such as a filter and an optical fiber).

### <Configuration of Detection Kit>

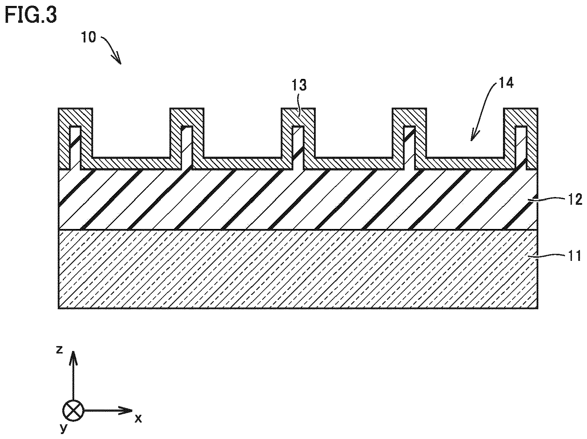

Fig. 2 is a conceptual diagram for illustrating the configuration of detection kit 10. Fig. 3 is a cross-sectional view of detection kit 10 taken along line III-III of Fig. 2. Detection kit 10 includes a substrate 11, a honeycomb polymer membrane 12, and thin film 13.

Substrate 11 provides mechanical strength to detection kit 10. In the present embodiment, detection kit 10 is irradiated with a laser beam from below, and thus a material transparent to the laser beam is used as a material of substrate 11. For example, glass can be adopted.

Honeycomb polymer membrane 12 is disposed on substrate 11. Honeycomb polymer membrane 12 is a polymer membrane having a plurality of pores 14 arranged in a honeycomb pattern along the surface of honeycomb polymer membrane 12. The pores 14 may be non-penetrating pores or penetrating pores communicating with adjacent pores. Resin (e.g., polystyrene) can be used as a material of honeycomb polymer membrane 12. See PTL 1 for description on a method for producing the honeycomb polymer membrane.

Thin film 13 is disposed on honeycomb polymer membrane 12. Thin film 13 may be formed on a part of a region to be irradiated with a laser beam (the position of a laser spot). In Embodiment 1, however, thin film 13 is formed to cover the entire surface of honeycomb polymer membrane 12. Thin film 13 has a thickness on the nanometer order. Thus, thin film 13 with the reflected structure of honeycomb polymer membrane 12 has a honeycomb structure.

Thin film 13 absorbs a laser beam from laser light source 50 and converts light energy into thermal energy. The material of thin film 13 is preferably a material having high light absorption (e.g., photothermal conversion efficiency) in the wavelength range of a laser beam (the near-infrared range in the present embodiment). In the present embodiment, a thin gold film is formed as thin film 13. Free electrons on a surface of the thin gold film form surface plasmon and are vibrated by a laser beam. This exhibits polarization. The energy of polarization is converted to the energy of lattice vibrations by Coulomb interaction between free electrons and an atomic nucleus. Consequently, the thin gold film generates heat. Hereinafter, this effect is also referred to as "photothermal effect."

The material of thin film 13 is not limited to gold and may be a metallic element (e.g., silver or platinum) capable of producing a photothermal effect other than gold or a metallic nanoparticle accumulated structure (e.g., a structure with a gold nanoparticle or a silver nanoparticle). Alternatively, the material of thin film 13 may be a material other than a metal and having high light absorption in the wavelength range of a laser beam. Such a material may be a material close to a black body (e.g., a carbon nanotube black body). Honeycomb polymer membrane 12 and thin film 13 correspond to "photothermal conversion region" according to the present disclosure.

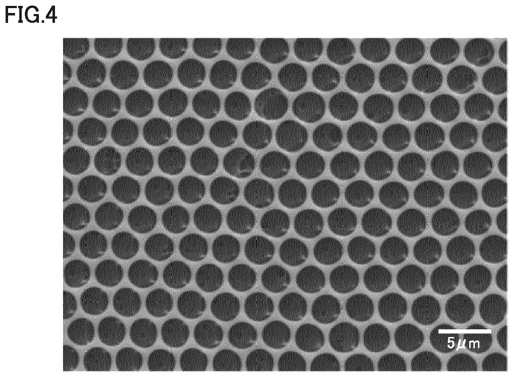

Fig. 4 is a diagram illustrating a top SEM (Scanning Electron Microscope) image of honeycomb polymer membrane 12 produced according to the present embodiment. Fig. 5 is a diagram illustrating a perspective SEM image of honeycomb polymer membrane 12 produced according to the present embodiment. In this example, pores 14 were 4 to 5 µm in diameter (pore diameter). Pores 14 were about 3 µm in depth.

### <Configuration of Microscopic Object>

In the present embodiment, a plurality of microscopic objects for detecting target bacteria are contained in a sample.

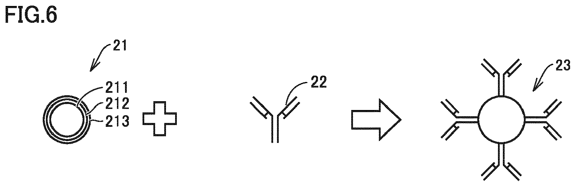

Fig. 6 is a diagram illustrating a microscopic object according to the present embodiment. In the example of Fig. 6, the microscopic object includes a magnetic bead 21. Magnetic bead 21 includes a core 211 of a high polymer, a magnetite layer 212 covering core 211, and a protective layer (for example, a hydrophilic polymer layer) 213 covering magnetite layer 212. Each of magnetite layer 212 and protective layer 213 may include two or more layers. As magnetic bead 21, for example, a Thermo Scientific Pierce Protein A/G magnetic bead (a particle diameter of 1 µm) of Thermo Fisher Scientific Inc. can be used. Magnetite layer 212 contains a magnetic substance (e.g., γFe₂O₃ and Fe₃O₄). Magnetic bead 21 exhibits paramagnetism. Thus, if an external magnetic field is not applied, magnetic beads 21 are dispersed in the sample.

The surface of magnetic bead 21 is modified by a host substance. In this example, the host substance is an antibody 22 that can be specifically bound to target bacteria. Various kinds of antibodies can be modified on the surface of the magnetic bead according to known techniques. Hereinafter, magnetic bead 21 modified by antibody 22 will be also described as "antibody-modified bead 23." Antibody-modified bead 23 is an example of "magnetic particle" according to the present disclosure.

Detection kit 10 being distributed to the market can be packaged in a container with liquid containing antibody-modified beads 23 with a predetermined concentration (in other words, in a wet state). In detection kit 10 to be distributed, most of antibody-modified beads 23 may be dispersed outside pores 14. In this case, antibody-modified beads 23 are introduced into pores 14 by a measurer prior to measurement. Alternatively, detection kit 10 may be distributed in a state in which most of antibody-modified beads 23 have been introduced into pores 14.

### <Introduction of Antibody-modified Bead>

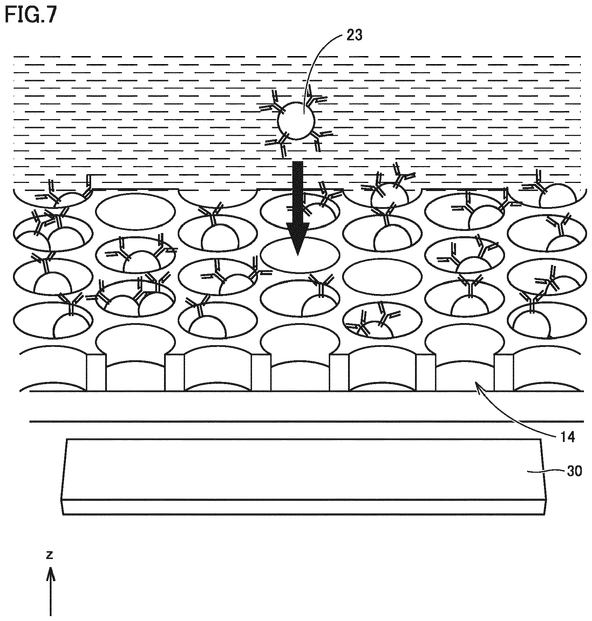

Fig. 7 is a conceptual diagram illustrating a state of antibody-modified beads 23 introduced into the plurality of pores 14 disposed on honeycomb polymer membrane 12. As illustrated in Fig. 7, when magnet 30 is disposed below detection kit 10, antibody-modified bead 23 is drawn downward by a magnetic field of magnet 30 and is introduced into pore 14.

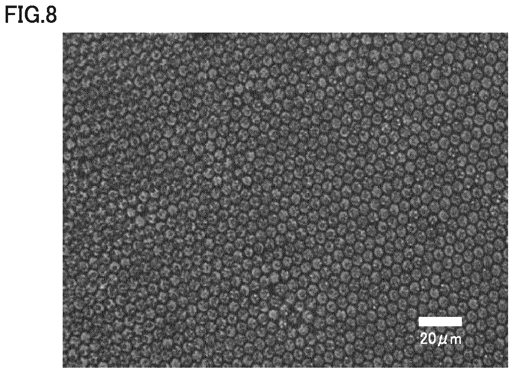

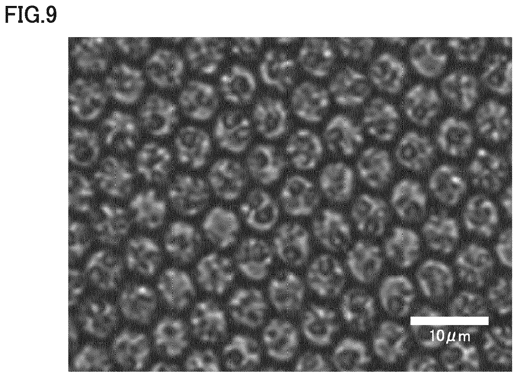

Fig. 8 is a diagram illustrating an optical transmission image of the top surface of detection kit 10 where antibody-modified beads 23 are introduced. Fig. 9 is an enlarged view of the optical transmission image shown in Fig. 8. For this measurement, objective lens 70 with a magnification of 100 times was used. Antibody-modified beads 23 (magnetic beads 21) were 1 µm in diameter. In other words, antibody-modified beads 23 are smaller than the pore diameter (4 to 5 µm). In this example, it was confirmed that about two or three antibody-modified beads 23 are trapped in each pore 14. Antibody-modified beads 23 are trapped thus in pores 14, so that antibody-modified beads 23 can be stably held on detection kit 10.

However, antibody-modified beads 23 do not necessarily have to be trapped. If antibody-modified beads 23 are larger than the pore diameter, antibody-modified beads 23 may be trapped while being placed on the opening portions of pores 14. Although antibody-modified beads 23 are trapped with lower stability than in trapping in pores 14, such a pattern of trapping can be also adopted.

How to introduce antibody-modified beads 23 into pores 14 is not limited to applying an external magnetic field to the sample by using magnet 30. For example, the sample may be irradiated with ultrasonic waves. Antibody-modified beads 23 in the sample can be introduced into pores 14 by the stirring effect with ultrasonic waves. Before a sample (not containing antibody-modified beads 23) is dropped, a liquid containing antibody-modified beads 23 can be dropped onto detection kit 10 to introduce antibody-modified beads 23 into pores 14 by using thermal convection (light concentration described later). Moreover, though it may take a longer time compared with using an external magnetic field, ultrasonic waves, or thermal convection, antibody-modified beads 23 can be also introduced into pores 14 by natural sedimentation or pipetting. This is because antibody-modified beads 23 have a larger specific gravity than the dispersion medium (typically, water) of the sample. If an external magnetic field is not used, microscopic objects (ordinary resin beads or metallic beads) that do not exhibit paramagnetism can be used instead of magnetic beads 21.

### <Detection of Bacteria>

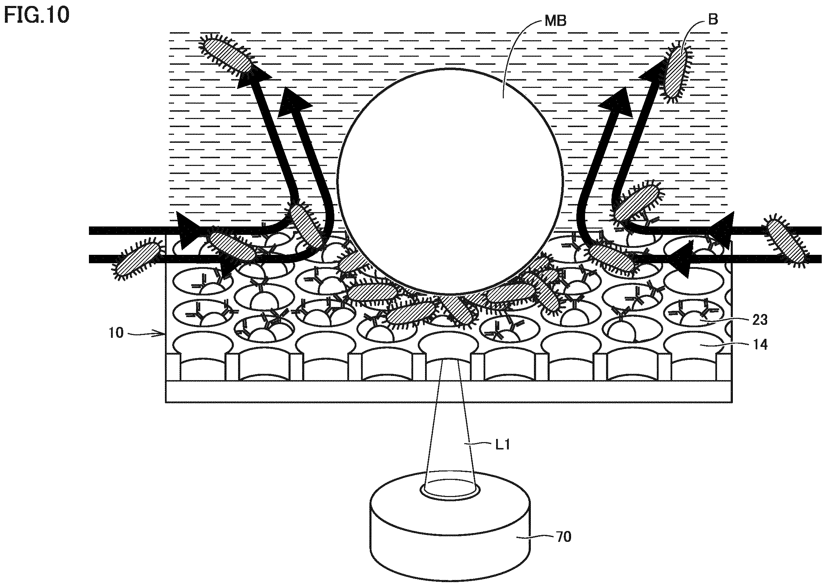

Fig. 10 is a conceptual diagram for illustrating a state of bacteria (denoted as B) accumulated by using detection kit 10. When starting the irradiation of detection kit 10 with a laser beam, by the photothermal effect of thin film 13 in the irradiation region of the laser beam (hereinafter also referred to as "laser spot"), the vicinity of the laser spot is locally heated. Thus, a microbubble (denoted as MB) is generated at the laser spot by boiling the dispersion medium (in this example, water) of a sample in the vicinity the laser spot, or the like. The microbubble grows with the passage of time.

With the irradiation with the laser beam, regular thermal convection constantly occurs in the dispersion medium in addition to the microbubble generation. As indicated by arrows, the direction of the thermal convection is temporarily directed to the microbubble and then is directed away from the microbubble. The reason for the occurrence of the thermal convection will be described below. Thermal convention is classified into buoyancy convection and Marangoni convection.

The closer to the laser spot, the higher the temperature of the dispersion medium. In other words, a temperature gradient is formed due to light irradiation in the dispersion medium. The temperature gradient generates buoyancy convection. More specifically, the dispersion medium above a region in which the microbubble is generated becomes relatively dilute by heating, and then the dispersion medium is floated by a buoyant force. Meanwhile, a dispersion medium having a relatively low temperature in the horizontal direction of the microbubble flows into the microbubble.

Generally, an interfacial tension generated on the surface of a bubble depends upon a molecular density on the surface of the bubble. The higher the molecular density, the smaller the interfacial tension. A molecular density in the present embodiment may be affected by the density of an analyte, in addition to the density of molecules forming the dispersion medium. Thus, in the presence of a density gradient of bacteria on the gas-liquid interface between the microbubble and the dispersion medium surrounding the microbubble, a region containing bacteria with a high density (typically, a lower region) is drawn toward a region containing bacteria with a low density (an upper region) such that the interfacial tension is balanced between the regions. At this timing, the movement of the gas-liquid interface at this moment propagates to a liquid (bulk) and generates Marangoni convection.

Bacteria are conveyed to the microbubble by thermal convection (buoyancy convection and/or Marangoni convection) and are trapped by the microbubble. More specifically, "stagnation region" in which the flow velocity of thermal convection is substantially 0 is formed between the microbubble and the bowl region. The bacteria conveyed by thermal convection are trapped into the stagnation region, so that the bacteria are accumulated near the laser spot. In this way, microbubble act as "stopper" for blocking bacteria and thus serve as an accumulation site of the bacteria. The concentrating effect that accumulates bacteria, which are dispersed in a sample, near a laser spot according to this mechanism can be also referred to as "light concentration."

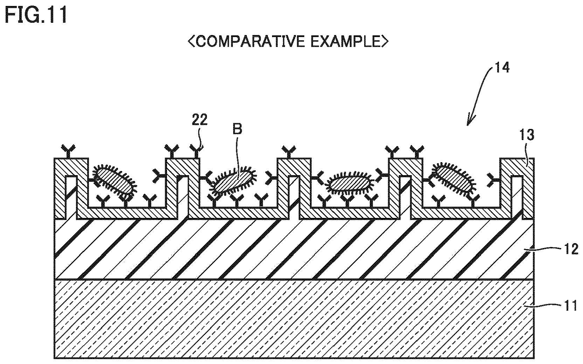

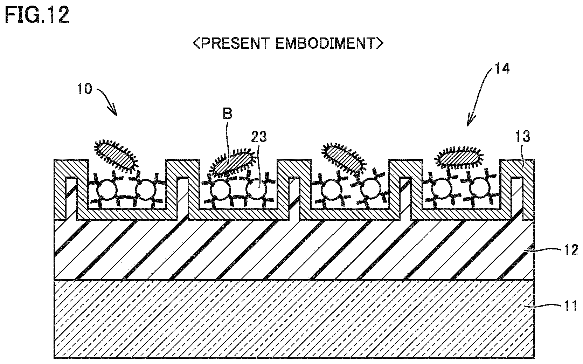

Fig. 11 is a diagram for explaining an accumulation pattern of bacteria in a comparative example. Fig. 12 is a diagram for explaining an accumulation pattern of bacteria in the present embodiment.

As illustrated in the comparative example, antibodies 22 may be directly modified on thin film 13. Then, however, only antibodies 22 are interposed between accumulated bacteria and thin film 13. Thus, heat generated on thin film 13 by the photothermal effect may be transmitted to bacteria near a laser spot and cause thermal damage to the bacteria. In that case, although bacteria are successfully accumulated with a high density, some of the bacteria near the laser spot are killed and thus the survival rate of all accumulated bacteria (= the number of living bacteria/the total number of accumulated bacteria) may be reduced.

In contrast, in the present embodiment, antibody-modified beads 23 with magnetic beads 21 serving as cores are interposed between bacteria and thin film 13. The thermal conductivity of magnetic beads 21 is considerably lower than that of thin film 13. As specific numeric values, whereas gold as a representative material of thin film 13 has a thermal conductivity of 320 [W/(m·K)], a polymer (e.g., polystyrene) as a core material of magnetic beads 21 has a thermal conductivity of about 0.1 [W/(m·K)]. As just described, the material having a low thermal conductivity is interposed between bacteria and thin film 13, so that a distance can be secured between bacteria and thin film 13 and thermal conduction from thin film 13 to bacteria can be suppressed. Thus, thermal damage to bacteria is reduced, thereby improving the survival rate of accumulated bacteria.

As illustrated in Figs. 2 to 4, partitions are formed between adjacent pores 14 of thin film 13. The optical system of detection system 2 is desirably adjusted so as to emit a laser beam to the partitions (particularly to the top surfaces of the partitions). The partitions protrude into a liquid, so that heat generated on the top surfaces of the partitions is intensively applied to liquid near the top surfaces of the partitions. Thus, a laser spot acts as "point heat source," which reduces the range of an excessive temperature increase around a laser spot. Furthermore, unlike in laser irradiation to the bottom surfaces of the pores, obstacles that may interfere with thermal convection are not present during laser irradiation to the partitions protruding into liquid. Thus, thermal convection can be generated with relatively low laser output power (see PTL 3 for a detailed description). As described above, with the adjustment of the laser spot to a proper position in addition to the introduction of antibody-modified beads 23, thermal damage to bacteria can be further reduced and the survival rate of bacteria can be further improved.

### <Detection Flow>

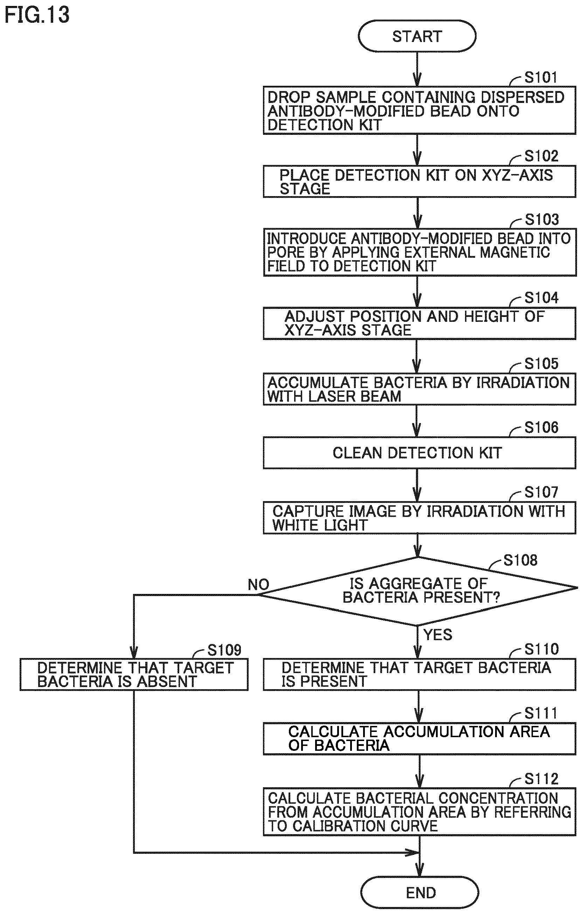

Fig. 13 is a flowchart indicating the steps of a method for detecting bacteria according to Embodiment 1. Flowcharts in Fig. 13 and Figs. 28 and 31, which will be described later, are executed when predetermined conditions are established (for example, when a measurer operates a measurement start button, which is not illustrated). The steps are basically implemented by software processing performed by controller 100. The steps may be implemented by hardware (electric circuit) disposed in controller 100. Hereinafter, the steps will be abbreviated as S.

In S101, a sample containing a plurality of dispersed antibody-modified beads 23 is prepared and is dropped onto detection kit 10. The amount of the dropped sample may be a small amount, for example, about several µL to several hundred µL but may be a larger amount. This processing may be performed by the measurer or may be automated by using a dispenser (not illustrated).

In S102, detection kit 10 is placed on xyz-axis stage 20. This processing may be performed by the measurer or may be automated by, for example, a mechanism (not illustrated) for feeding detection kit 10.

In S103, magnet 30 is disposed below detection kit 10. The measurer may manually bring magnet 30 close to detection kit 10 from below of detection kit 10. Alternatively, magnet 30 is placed on a movable stage (not illustrated) and controller 100 may control the movable stage so as to move magnet 30 to the beneath of detection kit 10. If magnet 30 is an electromagnet, magnet 30 may be configured to generate an external magnetic field by energization according to a command from controller 100. Antibody-modified beads 23 are introduced into pores 14 by applying an external magnetic field (see Fig. 7).

The processing of S103 may be performed before the processing of S 102. Specifically, detection kit 10 may be placed on xyz-axis stage 20 after antibody-modified beads 23 are introduced into pores 14.

In S104, the position of xyz-axis stage 20 is adjusted in the horizontal direction (x direction, y direction) and the vertical direction (z direction) such that a laser beam from laser light source 50 is emitted to the sample. This processing may be implemented by a manual operation of adjusting mechanism 40 by the measurer. Alternatively, this processing may be implemented by controlling adjusting mechanism 40 by controller 100. Positioning in the horizontal direction can be implemented by extracting the outer shape of the sample from an image captured by imaging device 91 using an image processing technique for pattern recognition. Moreover, the position of the beam waist of a laser beam in the vertical direction is known from the wavelength of the laser beam and the specifications (such as a magnification) of objective lens 70. Thus, the beam waist can be set to a target height by adjusting the position of xyz-axis stage 20 in the vertical direction.

In S105, controller 100 controls laser light source 50 so as to irradiate the sample with a laser beam for a predetermined time. The power of the laser beam (laser output power) and the irradiation time (laser irradiation time) are determined according to, for example, the specifications of detection kit 10 (such as the material and thickness of thin film 13) and the characteristics of bacteria (such as an assumed concentration and the size) on the basis of the results of previously conducted experiments or simulations. The laser output power and the laser irradiation time are desirably set to be large/long enough to generate a microbubble and convection in the sample and small/short enough to prevent excessive thermal damage to bacteria. Typically, the laser output power is several mW to several tens of mW and the laser irradiation time is several tens of seconds to several minutes. By the irradiation with the laser beam, bacteria are accumulated at the laser spot according to the mechanism illustrated in Fig. 10.

In S106, detection kit 10 is cleaned by the measurer. Thus, target bacteria specifically bound to antibody-modified beads are left on detection kit 10 and other bacteria are washed away. Cleaned detection kit 10 is placed on xyz-axis stage 20 again.

In S107, controller 100 controls illumination light source 80 to generate white light for illuminating detection kit 10 and controls imaging device 91 to capture an image of detection kit 10.

In S108, controller 100 determines whether an aggregate of bacteria is observed in the image captured by imaging device 91. If an aggregate of bacteria is not observed (NO at S107), controller 100 determines that the sample does not contain target bacteria (the concentration of bacteria in the sample is lower than the detection limit) (S109).

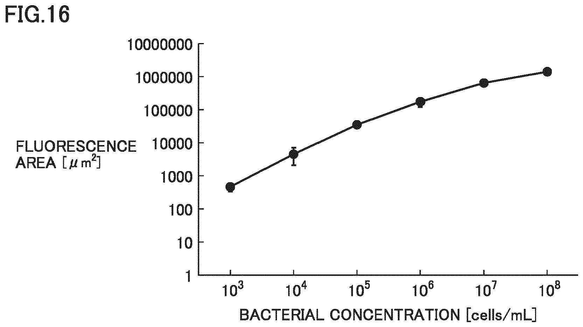

If an aggregate of bacteria is observed (YES at S108), controller 100 determines that the sample contains target bacteria (S110). In this case, controller 100 calculates the area (accumulation area) of a region where bacteria are accumulated (S111). Controller 100 can calculate the area of the region where bacteria are accumulated, for example, by extracting the region according to an image processing technique for pattern recognition. Furthermore, referring to a predetermined calibration curve (see Fig. 16), controller 100 calculates the concentration of bacteria (bacterial concentration) contained in the sample, from the accumulation area calculated in S111 (S112). At the completion of the processing of S 109 or S112, controller 100 returns the processing to the main routine.

### <Example>

### «1. Description of Fluorescent Image and Fluorescence Area»

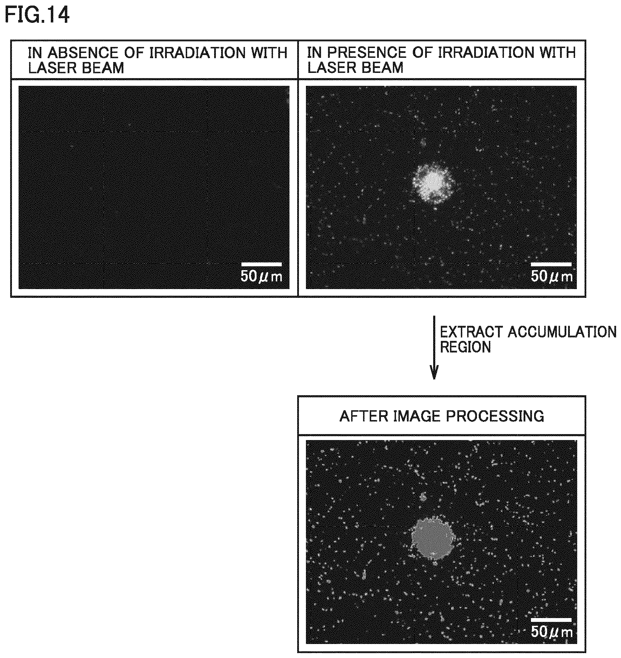

Fig. 14 is a diagram illustrating the images of detection kit 10 in the presence/absence of irradiation with a laser beam at a wavelength of 1064 nm and the extraction result of an area where bacteria are accumulated. In this example, E. coli (Escherichia coli) was used as target bacteria. Bacteria were subjected to fluorescent dyeing to capture a fluorescent image. The laser output power after passage through objective lens 70 (output power before passage through the objective lens) was set at 30 mW, and the laser irradiation time was set at three minutes. For image processing for calculating a fluorescence area (the accumulation area of target bacteria), commercial software (NIS-Elements of Nikon Corporation) was used. As shown in Fig. 14, it is confirmed that bacteria can be accumulated by irradiation with a laser beam and a fluorescence area can be calculated by image processing. In the absence of irradiation with a laser beam, the accumulation of bacteria was not confirmed after an equal time period. Thus, the result indicates that target bacteria can be more quickly detected by light concentration.

### «2. Concentration Dependence»

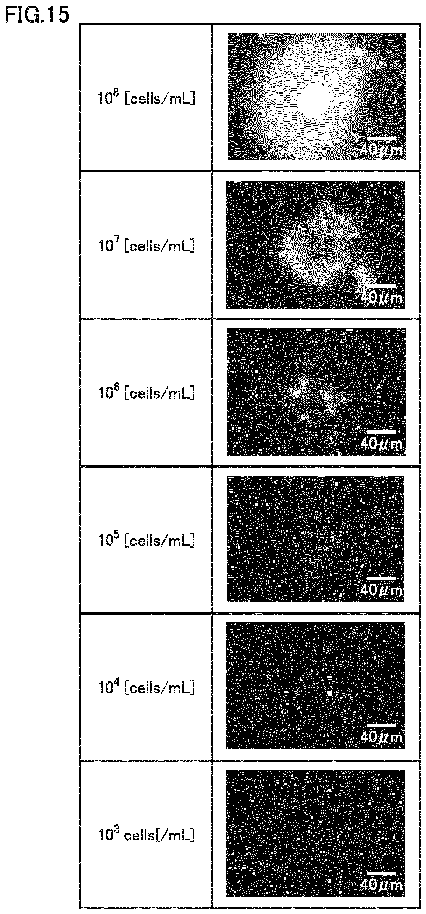

Fig. 15 is a diagram illustrating the accumulation results of bacteria in samples with different bacterial concentrations. E. coli was used as target bacteria. Six samples with different bacterial concentrations ranging from 10³[cells/mL] to 10⁸[cells/mL] were prepared. Also in this example, bacteria were subjected to fluorescent dyeing to capture a fluorescent image. The laser output power was set at 20 mW, and the laser irradiation time was set at seven minutes. Fluorescence (that is, the accumulation of bacteria) was confirmed also in the sample having the lowest bacterial concentration of 10³[cells/mL].

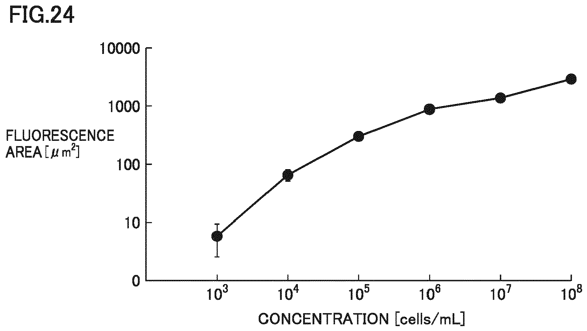

Fig. 16 is a diagram illustrating the correlation between a bacterial concentration and a fluorescence area, the correlation being determined from the images shown in Fig. 15. The horizontal axis indicates a bacterial concentration on a logarithmic scale. The vertical axis indicates a fluorescence area (accumulation area of bacteria) on a logarithmic scale. Three measurements were conducted at each bacterial concentration. This also applies to Fig. 24 (described later). As shown in Fig. 16, the correlation between a bacterial concentration and a fluorescence area is linearly expressed on a log-log graph. Such a correlation is determined in advance and is used as a calibration curve, thereby enabling to calculate a bacterial concentration from a fluorescence area (see S112 of Fig. 13).

### «3. Selective Detection»

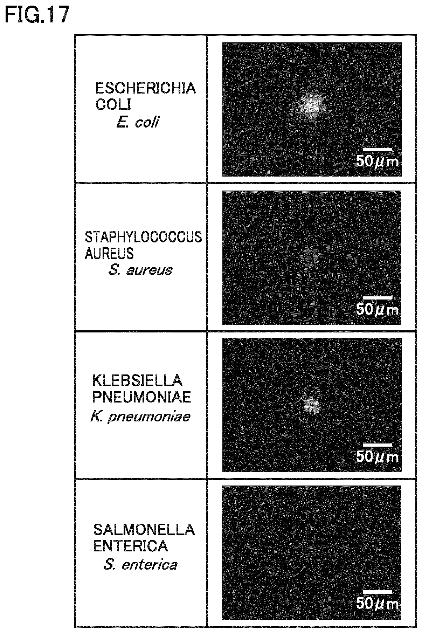

Fig. 17 is a diagram illustrating the accumulation results of various kinds of bacteria. In this example, four samples were prepared to accumulate four kinds of bacteria. Specifically, S. aureus (Staphylococcus aureus), K. pneumoniae (Klebsiella pneumoniae), and S. enterica (Salmonella enterica) were used in addition to E. coli as target bacteria. The bacteria were subjected to fluorescent dyeing. In this example, each of the samples contains only one kind of bacteria. A bacterial concentration in each of the samples was 10⁸[cells/mL]. The laser output power was set at 30 mW, and the laser irradiation time was set at three minutes. Antibody 22 specifically bound to E. coli was used for antibody-modified beads 23. In this case, circular fluorescence was observed even after cleaning, though specific binding to antibody-modified beads 23 does not occur in bacteria other than E. coli. This is because if densely accumulated, the nonspecific adsorption is not negligible even for bacteria other than E. coli.

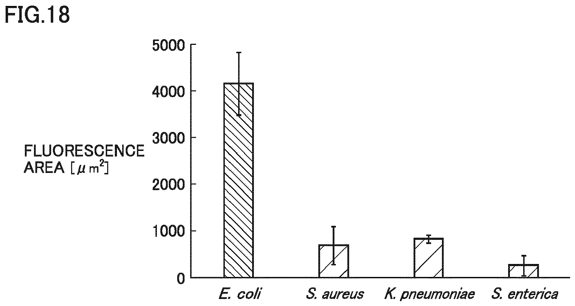

Fig. 18 is a diagram illustrating the calculation result of a fluorescence area (accumulation area) for each kind of bacteria, the calculation result being determined from the images shown in Fig. 17. An error bar indicates a standard deviation in three measurements. The accumulation area of E. coli was about 5 to 15 times larger than those of three other kinds of bacteria. Such a considerable difference in accumulation area can definitely discriminate E. coli from other bacteria, suggesting that selective detection (specific detection) of E. coli was successful.

### «4. Mixed Bacteria Sample»

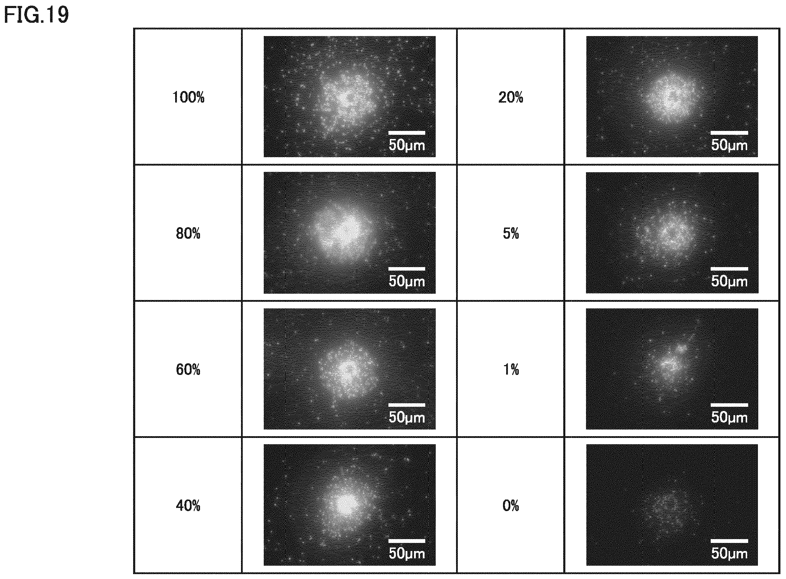

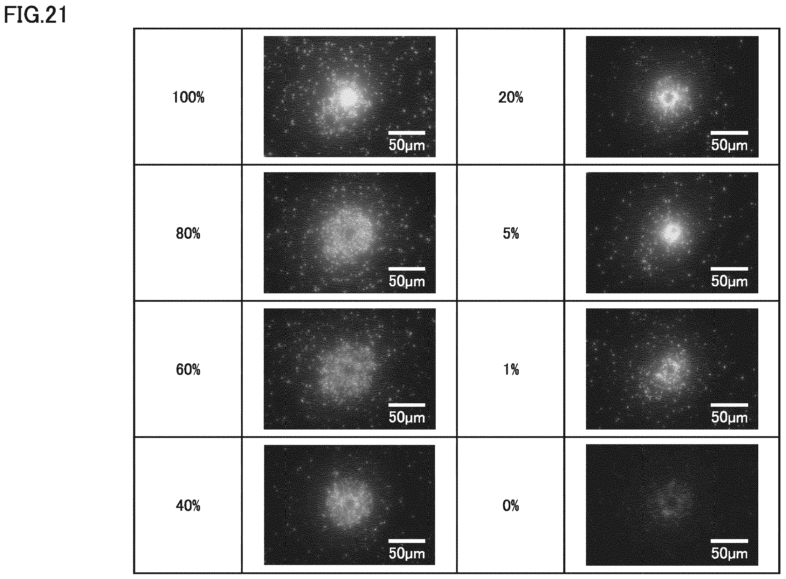

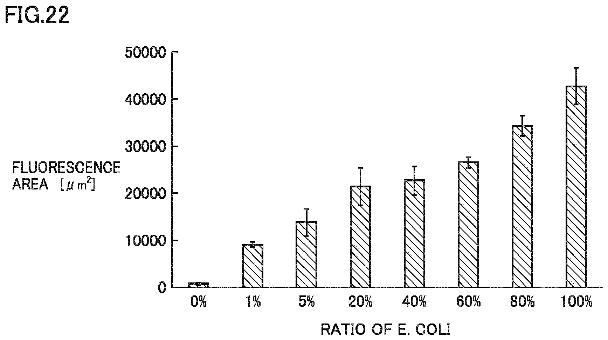

Fig. 19 is a diagram illustrating the accumulation results of bacteria in mixed bacteria samples containing two kinds of bacteria. In this measurement, the mixed bacteria samples containing E. coli and S. aureus were used. E. coli and S. aureus were both subjected to fluorescent dyeing. Eight samples in which the ratio of E. coli (= the number of contained E. coli/total number of bacteria) varies from 0% to 100% were prepared. The total concentration of two kinds of bacteria in each sample was 10⁸[cells/mL]. The laser output power (output power before passage through the objective lens) was set at 20 mW, and the laser irradiation time was set at seven minutes. Antibody 22 specifically bound to E. coli was used for antibody-modified beads 23. These conditions are also shared in Figs. 21 and 22, which will be described later. As shown in Fig. 19, it was observed that the fluorescence area tends to increase with the ratio of E. coli.

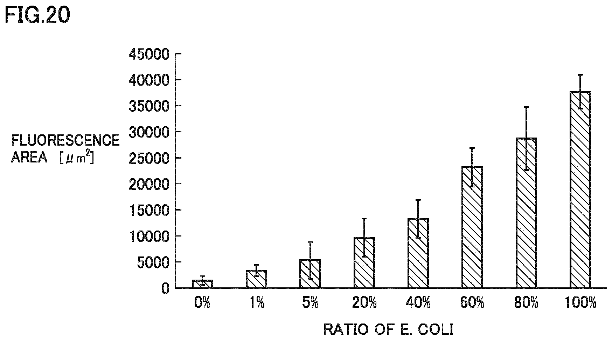

Fig. 20 is a diagram illustrating the calculation result of a fluorescence area, the calculation result being determined from the images shown in Fig. 19. The horizontal axis indicates the ratio of E. coli. The vertical axis indicates a fluorescence area (accumulation area of E. coli). An error bar indicates a standard deviation in three measurement results. The number of measurements was three (n = 3). This also applies to Fig. 22 (described later). As shown in Fig. 20, it was confirmed that the fluorescence area monotonously increases with the ratio of E. coli. This suggests that the selective detection of E. coli was successful in the mixed bacteria samples containing two kinds of bacteria.

Fig. 21 is a diagram illustrating the accumulation results of bacteria in mixed bacteria samples containing four kinds of bacteria. In this measurement, the mixed bacteria samples containing E. coli, S. aureus, K. pneumoniae, and S. enterica were used. All bacteria including E. coli, S. aureus, K. pneumoniae, and S. enterica were subjected to fluorescent dyeing. Eight samples in which the ratio of E. coli varies from 0% to 100% were prepared. The total concentration of four kinds of bacteria in each sample was 10⁸[cells/mL]. Also in this measurement, it was observed that the fluorescence area tends to increase with the ratio of E. coli.

Fig. 22 is a diagram illustrating the calculation result of a fluorescence area, the calculation result being determined from the images shown in Fig. 21. It was confirmed that the fluorescence area monotonously increases with the ratio of E. coli. Thus, the selective detection of E. coli was successful also in the mixed bacteria samples containing four kinds of bacteria.

### «5. Impurity Sample»

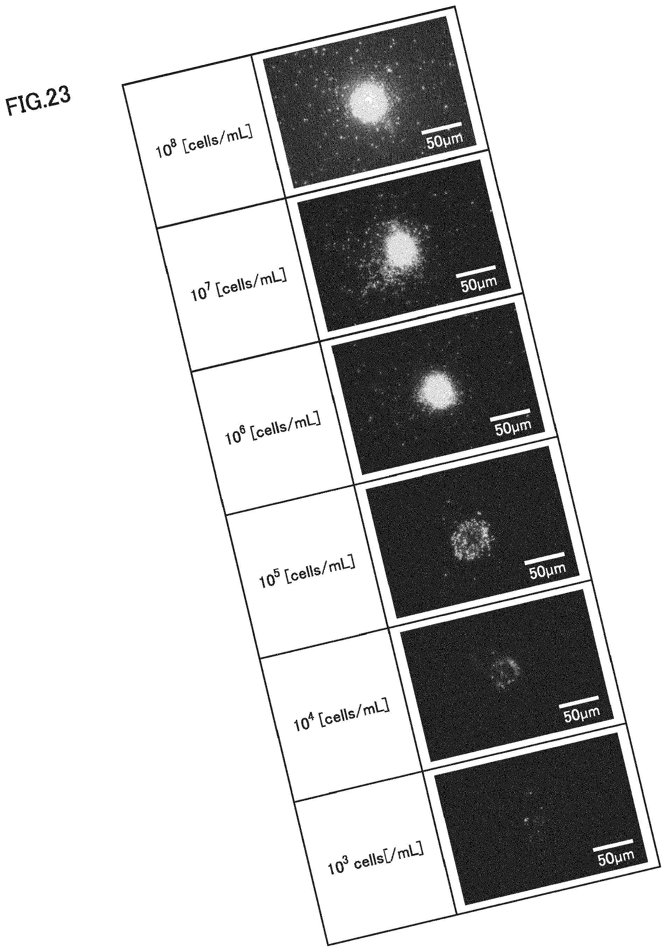

Fig. 23 is a diagram illustrating the accumulation results of bacteria in impurity samples. The impurity sample is a sample containing, in addition to an analyte, a substance other than the analyte as an impurity. In this measurement, the impurity samples containing E. coli in apple juice were used. More specifically, bacteria with a concentration series indicated below were added (standard addition) to apple juice, and then the apple juice was left at rest for ten minutes. Furthermore, the bacteria were dispersed again into a phosphate buffer after centrifugation. As in Figs. 15 and 16, six samples with different bacterial concentrations from 10³[cells/mL] to 10⁸[cells/mL] were prepared. Also in this example, bacteria were subjected to fluorescent dyeing to capture a fluorescent image. The laser output power was set at 20 mW, and the laser irradiation time was set at seven minutes. Also in apple juice containing impurities, fluorescence was confirmed in the sample having the lowest bacterial concentration of 10³[cells/mL].

Fig. 24 is a diagram illustrating the correlation between a bacterial concentration and a fluorescence area, the correlation being determined from the images shown in Fig. 23. The number of measurements was three (n = 3). As in Fig. 16, the correlation between a bacterial concentration and a fluorescence area is almost linearly expressed on a log-log graph. Such a calibration curve is prepared, thereby enabling to calculate a bacterial concentration from a fluorescence area. In many cases, food poisoning is caused by offending bacteria thriving in a food item or beverage containing impurities. In some cases, food poisoning is caused by bacteria in impurities attached to tableware (such as a plate and a glass) or cookware (such as a chopping board and a kitchen knife). The results of Figs. 23 and 24 suggest successful detection under conditions close to an actual environment. In the case of a food item, conventionally, a bacteriological examination is typically conduced in impurities contained in a supernatant after shaking with a stomacher or the like.

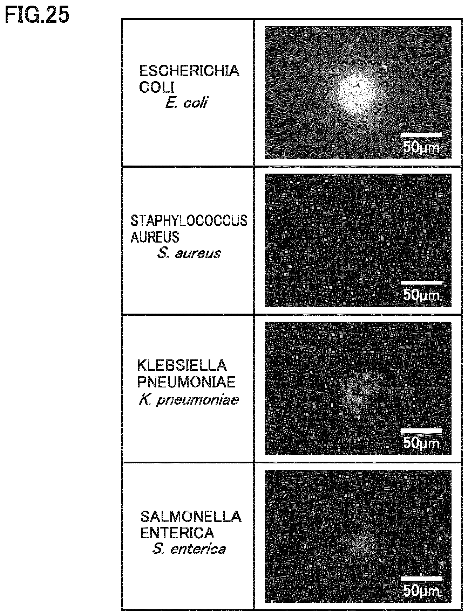

Fig. 25 is a diagram illustrating the accumulation results of various kinds of bacteria in impurity samples. As in Fig. 17, four samples were prepared. Each of the samples contains any one of four kinds of bacteria (E. coli, S. aureus, K. pneumoniae, and S. enterica). The bacteria were subjected to fluorescent dyeing. Antibody 22 specifically bound to E. coli was used for antibody-modified beads 23. A bacterial concentration in each of the samples was 10⁸[cells/mL]. The laser output power was set at 20 mW, and the laser irradiation time was set at seven minutes. Although fluorescence was observed in all of the four samples, the sample containing E. coli showed the most intensive fluorescence.

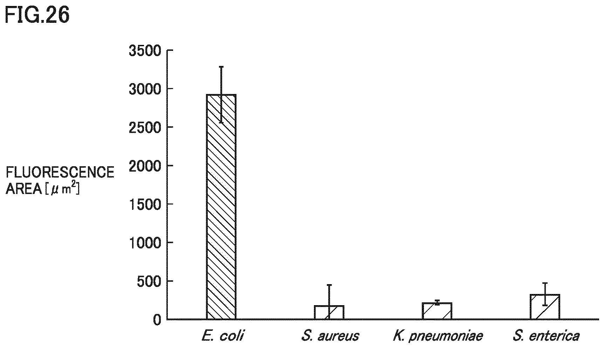

Fig. 26 is a diagram illustrating the calculation result of a fluorescence area for each kind of bacteria, the calculation result being determined from the images shown in Fig. 25. An error bar indicates a standard deviation in three measurement results. The fluorescence area of E. coli was 8 to 15 times larger than those of three other kinds of bacteria. Thus, the selective detection of E. coli was successful also in apple juice containing impurities.

As described above, in Embodiment 1, bacteria are accumulated and detected by using detection kit 10 with antibody-modified beads 23 introduced into pores 14. Antibody 22 specifically bound to target bacteria was modified on the surface of antibody-modified bead 23, so that target bacteria can be selectively detected. Moreover, bacteria can be accumulated with high efficiency by using thermal convection generated by the photothermal effect resulting from irradiation with a laser beam, thereby reducing the accumulation time to several tens of seconds to several minutes. Hence, according to Embodiment 1, an analyte that may be contained in a sample can be selectively and quickly detected with high sensitivity.

### [Embodiment 2]

In Embodiment 2, a configuration for detecting target bacteria on the basis of the spectrum of a detection kit 10 will be described.

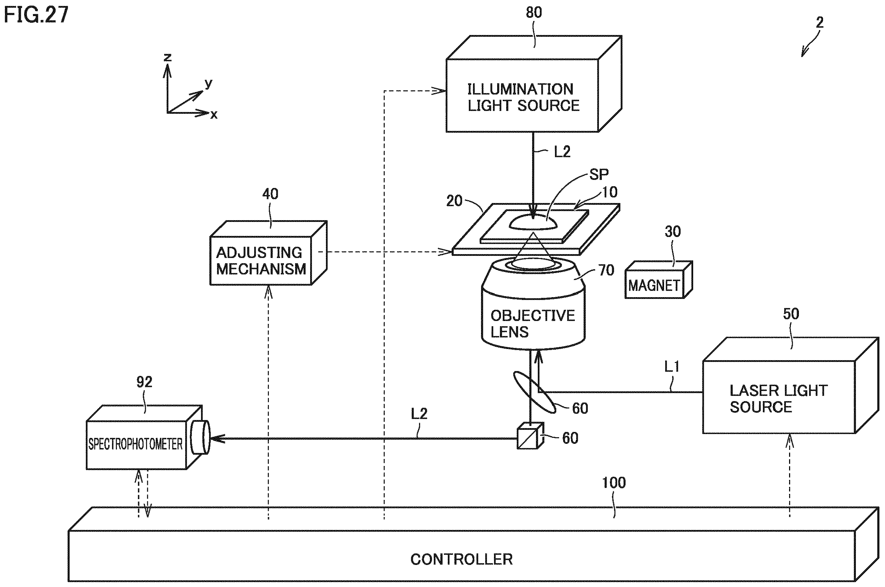

Fig. 27 is a diagram of the overall configuration of a detection system 2 of bacteria according to Embodiment 2. Detection system 2 is different from detection system 1 (see Fig. 1) according to Embodiment 1 in that a spectrophotometer 92 is provided instead of an imaging device 91.

Spectrophotometer 92 measures the reflection spectrum of detection kit 10 according to a command from a controller 100 and outputs the result of measurement to controller 100. Spectrophotometer 92 includes, for example, a diffraction grating, a light receiving element, a shutter, and a slit (none of them are illustrated). Light incident on spectrophotometer 92 passes through the slit and then reaches the diffraction grating. In the diffraction grating, incident light is reflected in a direction corresponding to the wavelength. The surface of the light receiving element is divided into a plurality of unit regions. Light reflected by the diffraction grating enters the unit region corresponding to the wavelength among the plurality of unit regions of the light receiving element. Thereafter, the reflection spectrum is acquired on the basis of an intensity value in each of the unit regions. Spectrophotometer 92 is preferably capable of measuring a reflection spectrum in a wavelength range (for example, a wavelength range from a visible range to a near-infrared range) wider than the absorption wavelength range of a thin film 13. Moreover, spectrophotometer 92 preferably has wavelength resolution as small as possible. The wavelength resolution of spectrophotometer 92 is, for example, 10 nm or less, 5 nm or less, 2 nm or less, or 1 nm or less and is not limited thereto. Spectrophotometer 92 corresponds to "light receiver," "spectroscope," and "detector" according to the present disclosure.

Configurations other than spectrophotometer 92 in detection system 2 are equivalent to the corresponding configurations of detection system 1 and thus a description thereof is not repeated. Fig. 27 illustrates an optical system for measuring the transmission spectrum of detection kit 10. Detection system 2 may be configured to measure other spectrums (including a reflection spectrum and a scattering spectrum).

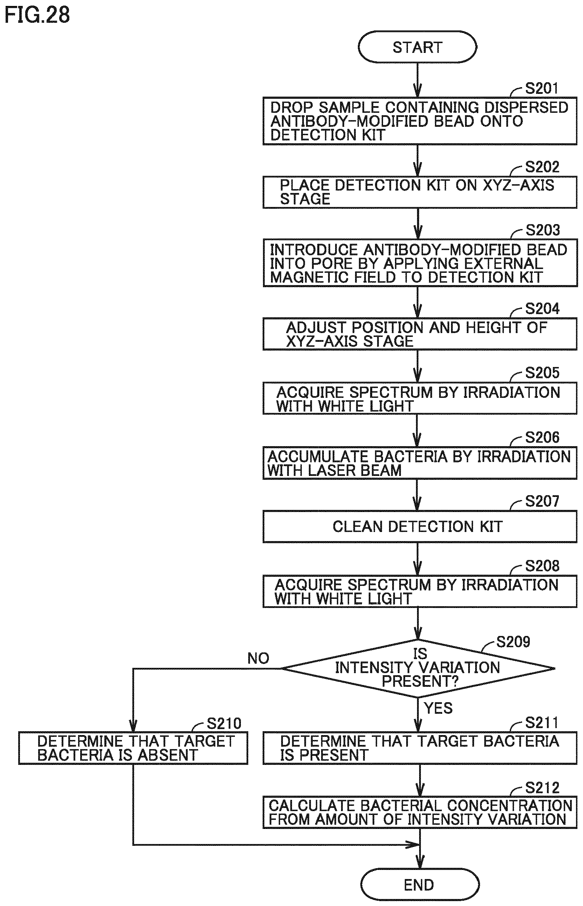

Fig. 28 is a flowchart indicating the steps of a method for detecting bacteria according to Embodiment 2. The processing of S201 to S204 is similar to the processing of S101 to S104 (see Fig. 13) in Embodiment 1.

In S205, controller 100 controls an illumination light source 80 so as to emit white light. Controller 100 then acquires, from spectrophotometer 92, the transmission spectrum of a region to be irradiated with a laser beam before the irradiation with the laser beam.

In S206, controller 100 controls a laser light source 50 so as to output a laser beam with predetermined power for a predetermined time. After bacteria are accumulated, the irradiation with the laser beam is stopped. Thereafter, detection kit 10 is cleaned by a measurer to wash off bacteria other than target bacteria (S207). Cleaned detection kit 10 is placed on an xyz-axis stage 20 again.

In S208, controller 100 acquires, from spectrophotometer 92, the transmission spectrum of the region irradiated with a laser beam. After the acquisition of the transmission spectrum, the irradiation with white light can be terminated. The kind of spectrum acquired in detection system 2 is not particularly limited. Detection system 2 may acquire a reflection spectrum or an extinction spectrum or acquire a fluorescence spectrum.

In S209, controller 100 determines the presence or absence of an intensity variation of the transmission spectrum by comparing the transmission spectrum acquired in S205 and the transmission spectrum acquired in S208. For example, controller 100 can determine the presence of an intensity variation when an intensity variation equal to or larger than a predetermined amount is detected at a predetermined specific wavelength. If an intensity variation of the transmission spectrum is not detected (NO at S209), controller 100 determines that the sample does not contain target bacteria (S210).

If an intensity variation of the transmission spectrum is detected (YES at S209), controller 100 determines that the sample contains target bacteria (S211). Controller 100 then calculates the concentration of the target bacteria from the amount of intensity variation (S212). Like the calibration curve described in Fig. 16, the processing is also implemented by determining the correlation between the amount of intensity variation and the bacterial concentration at a specific wavelength in advance. At the completion of the processing of S210 or S212, controller 100 returns the processing to the main routine.

As described above, also in Embodiment 2, bacteria are accumulated by using detection kit 10 with antibody-modified beads 23 introduced into pores 14. Thus, target bacteria can be selectively accumulated with high efficiency. In the detection of target bacteria, a spectrum is used instead of the accumulation area of bacteria. Also in this case, the target bacteria can be selectively detected as in Embodiment 1. Hence, according to Embodiment 2, an analyte that may be contained in a sample can be selectively and quickly detected.

### [Embodiment 3]

In Embodiment 3, a configuration for detecting target bacteria on the basis of an electric resistance (impedance) of a detection kit will be described.

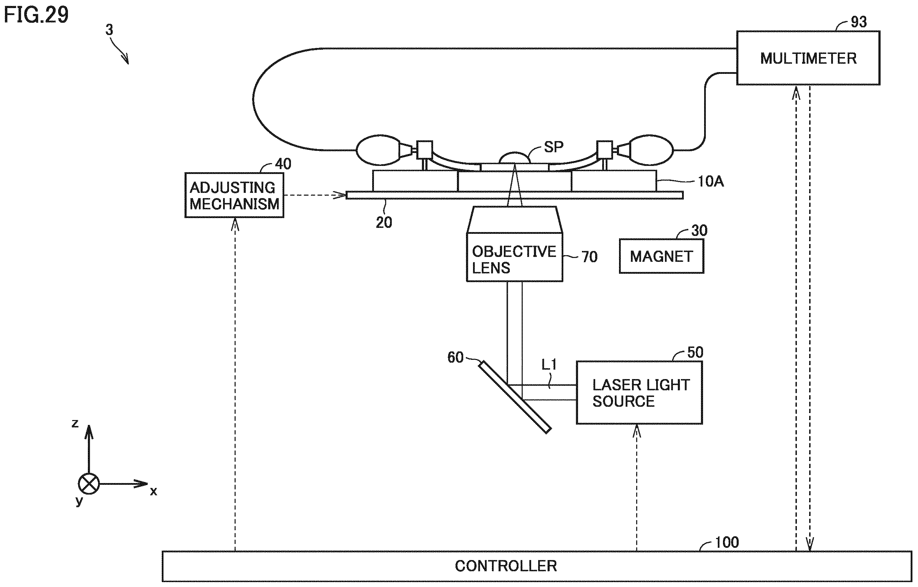

Fig. 29 is a diagram of the overall configuration of a detection system 3 of bacteria according to Embodiment 3. Detection system 3 is different from detection system 1 (see Fig. 1) according to Embodiment 1 in that a detection kit 10A is provided instead of detection kit 10 and a multimeter 93 is provided instead of illumination light source 80 and imaging device 91.

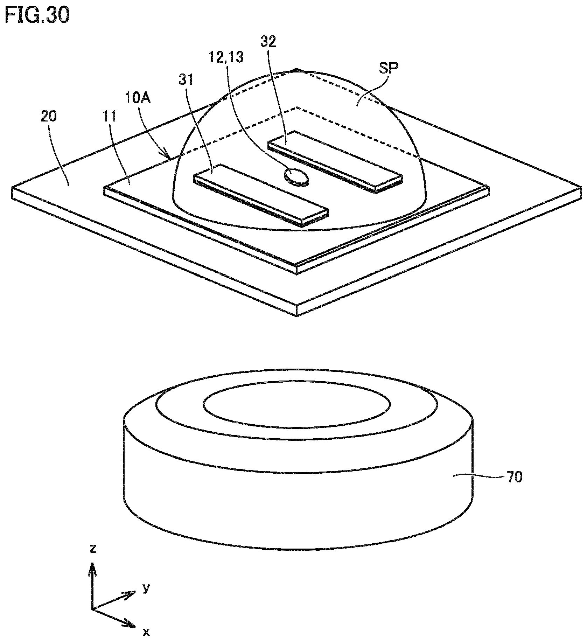

Multimeter 93 is an impedance measuring device configured to measure an electric resistance between an electrode 31 and an electrode 32 (see Fig. 30) that are provided on detection kit 10A. More specifically, multimeter 93 measures a voltage between electrode 31 and electrode 32 while applying a constant current between electrode 31 and electrode 32 according to a command from a controller 100, for example. The result of measurement by multimeter 93 is outputted to controller 100. Multimeter 93 is another example of "detector" according to the present disclosure.

In this example, a configuration for measuring a voltage between electrodes 31 and 32 while performing constant current control by multimeter 93, that is, a configuration in which multimeter 93 acts as a galvanostat will be described. However, a potentiostat may be used instead of multimeter 93. If a potentiostat is used, a current passing between electrodes 31 and 32 is measured at the application of a constant voltage between electrodes 31 and 32.

Fig. 30 is a diagram for explaining the detail of the configuration of detection kit 10A according to Embodiment 3. Detection kit 10A further includes electrodes 31 and 32 in addition to a substrate 11, a honeycomb polymer membrane 12, and a thin film 13.

Electrodes 31 and 32 are disposed on substrate 11. Electrode 31 is an anode, and electrode 32 is a cathode. Each of electrodes 31 and 32 is a metallic thin film having a film thickness on the nanometer order, for example, a thin platinum film. An adhesive layer (e.g., a thin titanium film) for increasing adhesion between detection kit 10 and electrode 31 may be disposed between substrate 11 and electrode 31. This applies also to electrode 32.

Electrode 31 and electrode 32 are arranged apart from each other with honeycomb polymer membrane 12 and thin film 13 interposed therebetween. By the irradiation with a laser beam, target bacteria are accumulated between electrode 31 and electrode 32. As target bacteria are accumulated, a cross-link is formed between electrode 31 and electrode 32 at a certain point of time by target bacteria. Thus, the main component of an electric resistance measured by multimeter 93 changes from an electric resistance of a dispersion medium of a sample to an electric resistance of target bacteria accumulated between electrode 31 and electrode 32. If a liquid (e.g., water) having sufficiently high insulation resistance is used as a dispersion medium, the electric resistivity of target bacteria is lower than that of the dispersion medium. Thus, if a reduction in electric resistance is detected, it can be determined that a cross-link is formed between electrode 31 and electrode 32 by target bacteria, in other words, target bacteria are detected. See PTL 2 for a detailed description on a mechanism for measuring electrical characteristics between electrodes 31 and 32. Electrode 31 and electrode 32 correspond to "first electrode" and "second electrode" according to the present disclosure.

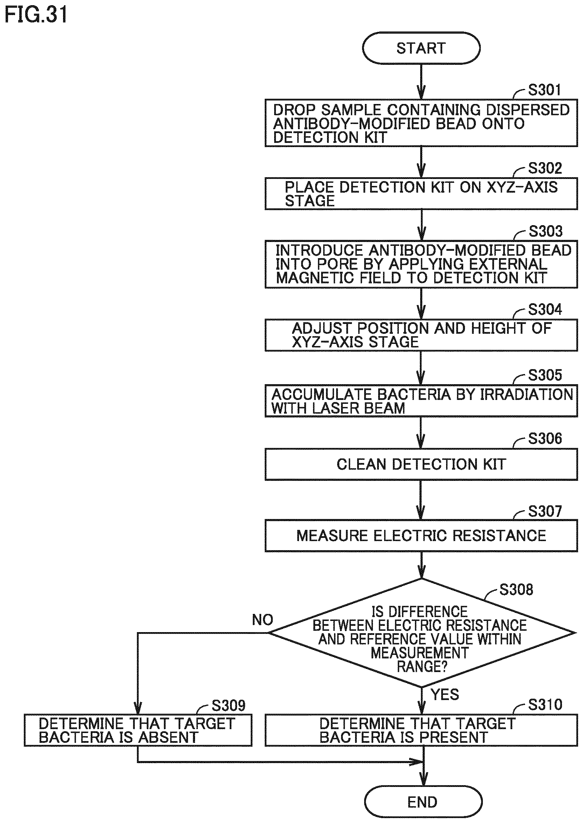

Fig. 31 is a flowchart indicating the steps of a method for detecting bacteria according to Embodiment 3. The processing of S303 to S306 is similar to the processing of S101 to S106 (see Fig. 13) in Embodiment 1 and thus a description thereof is not repeated.

In S307, controller 100 acquires, from multimeter 93, a voltage between electrode 31 and electrode 32 on cleaned detection kit 10A. Controller 100 then calculates an electric resistance between electrode 31 and electrode 32.

In S308, controller 100 determines whether a difference (absolute value) between the electric resistance calculated in S307 and a reference value falls within a predetermined measurement range corresponding to the characteristics of multimeter 93. The reference value is set on the basis of the electric resistance of a sample (dispersion medium) that does not contain target bacteria.

If the difference falls outside the measurement range (NO at S308), controller 100 determines that the sample does not contain target bacteria (S309). If the difference falls within the measurement range (YES at S308), controller 100 determines that the sample contains target bacteria (S310). At the completion of the processing of S309 or S310, controller 100 returns the processing to the main routine.

As described above, also in Embodiment 3, bacteria are accumulated by using detection kit 10 with antibody-modified beads 23 introduced into pores 14. Thus, target bacteria can be selectively accumulated with high efficiency. Moreover, in Embodiment 3, a change of electric resistivity is used for detecting target bacteria. Also in this case, as in Embodiments 1 and 2, target bacteria can be selectively detected. Hence, according to Embodiment 3, an analyte that may be contained in a sample can be selectively and quickly detected in a sample.

### [Supplement]

Finally, the aspects of the present disclosure will be collectively described.

### (Supplement 1)

A method for detecting an analyte that may be contained in a liquid sample by using a detection kit,
the detection kit including a photothermal conversion region that absorbs light and converts the light into heat,
a plurality of pores being disposed in the photothermal conversion region,
the method including:
   introducing a plurality of microscopic objects into the plurality of pores, each of the plurality of microscopic objects having a surface modified by a host substance capable of being specifically bound to the analyte;
   heating the liquid sample to generate thermal convection in the liquid sample by irradiating the photothermal conversion region with light having a wavelength included in an absorption wavelength range of the photothermal conversion region; and
   detecting the analyte, by monitoring the detection kit after the irradiation with the light.

### (Supplement 2)

The method for detecting an analyte according to Supplement 1, wherein sizes of the plurality of microscopic objects are smaller than the pore diameters of the plurality of pores.

### (Supplement 3)

The method for detecting an analyte according to Supplement 1 or 2, wherein the plurality of microscopic objects each include a core,
the photothermal conversion region includes a thin film that converts the light into heat, and
the core has a thermal conductivity lower than a thermal conductivity of the thin film.

### (Supplement 4)

The method for detecting an analyte according to any one of Supplements 1 to 3, wherein the plurality of pores are arranged in a honeycomb pattern.

### (Supplement 5)

The method for detecting an analyte according to any one of Supplements 1 to 4, wherein the plurality of microscopic objects each include a magnetic particle, and
the introducing includes introducing the plurality of microscopic objects into the plurality of pores by an external magnetic field.

### (Supplement 6)

The method for detecting an analyte according to any one of Supplements 1 to 4, wherein the introducing includes introducing the plurality of microscopic objects into the plurality of pores by irradiation with ultrasonic waves.

### (Supplement 7)

The method for detecting an analyte according to any one of Supplements 1 to 4, wherein the introducing includes introducing the plurality of microscopic objects into the plurality of pores by thermal convection generated by irradiating the photothermal conversion region with the light.

### (Supplement 8)

The method for detecting an analyte according to any one of Supplements 1 to 4, wherein the plurality of microscopic objects are larger than the liquid sample in specific gravity, and
the introducing includes introducing the plurality of microscopic objects into the plurality of pores by natural sedimentation of the plurality of microscopic objects.

### (Supplement 9)

The method for detecting an analyte according to any one of Supplements 1 to 8, wherein the detecting the analyte includes:
detecting, by a receiver, light from the liquid sample after irradiation with the light; and
calculating a concentration of the analyte in the liquid sample, on the basis of a signal from the receiver.

### (Supplement 10)

The method for detecting an analyte according to Supplement 9, wherein the receiver includes an imaging device, and
the calculating the concentration of the analyte includes:
calculating an accumulation area of the analyte, from an image captured by the imaging device; and
calculating the concentration of the analyte, from the calculated accumulation area by referring to the correlation between the concentration of the analyte and the accumulation area of the analyte.

### (Supplement 11)

The method for detecting an analyte according to Supplement 10, wherein the analyte is labeled with a fluorescent dye, and
the calculating the accumulation area of the analyte includes calculating a fluorescence area.

### (Supplement 12)

The method for detecting an analyte according to any one of Supplements 1 to 11, wherein the detecting the analyte includes selectively detecting the analyte, from a plurality of substances including the analyte.

### (Supplement 13)

The method for detecting an analyte according to any one of Supplements 1 to 12, wherein the liquid sample is a sample containing an impurity, and
the detecting the analyte includes detecting the analyte contained in the liquid sample containing the impurity.

### (Supplement 14)

The method for detecting an analyte according to Supplement 9, wherein the receiver includes a spectroscope that measures the spectrum of light from the liquid sample, and
calculating the concentration of the analyte includes calculating the concentration of the analyte, on the basis of the spectrum measured by the spectroscope.

### (Supplement 15)

The method for detecting an analyte according to any one of Supplements 1 to 8, wherein the detection kit further includes first and second electrodes being arranged apart from each other with the photothermal conversion region interposed between the electrodes, and
the detecting the analyte includes detecting the analyte, on the basis of a change of an electric resistance between the first electrode and the second electrode.

### (Supplement 16)

A detection kit for an analyte that may be contained in a liquid sample comprising:
a photothermal conversion region that absorbs light and converts the light into heat, a plurality of pores being disposed in the photothermal conversion region; and
a plurality of microscopic objects disposed in the plurality of pores, each of the plurality of microscopic objects having surfaces modified by a host substance capable of being specifically bound to the analyte.

### (Supplement 17)

A detection system for an analyte, including: a detection kit according to Supplement 16;
a light source that emits light having a wavelength within the absorption wavelength range of the photothermal conversion region; and
a detector that detects the analyte, by monitoring the detection kit after irradiation with the light from the light source.

### (Supplement 18)

A method for manufacturing a detection kit used for detecting an analyte that may be contained in a liquid sample, the method including:
preparing the detection kit including a photothermal conversion region in which a plurality of pores are disposed; and
introducing a plurality of microscopic objects into the plurality of pores, each of the plurality of microscopic objects having a surface modified by a host substance capable of being specifically bound to the analyte.

It should be understood that the disclosed embodiments are merely exemplary and are not restrictive in all the aspects. The scope of the present disclosure is not indicated by the description of the embodiments but the claims. The scope of the present disclosure is intended to include meanings equivalent to the claims and all changes in the scope thereof.

### INDUSTRIAL APPLICABILITY

The present disclosure is usable in a form of quickly detecting an analyte with high sensitivity by accumulating various analytes (e.g., toxic fine particles such as PM2.5, environmental load substances such as microplastics or nano-plastics, or various bacteria or viruses) with high efficiency, the substances being dispersed in a liquid sample. Furthermore, the present disclosure is also usable for determining whether microscopic objects are included in a liquid sample and/or specifying the concentration of microscopic objects in a liquid sample.

### REFERENCE SIGNS LIST

1 to 3 Detection system; 10, 10A Detection kit; 11 Substrate; 12 Honeycomb polymer membrane; 13 Thin film; 14 Pore; 20 Xyz-axis stage; 21 Magnetic bead; 211 Core; 212 Magnetite layer; 213 Protective layer; 22 Antibody; 23 Antibody-modified bead; 30 Magnet; 31,32 Electrode; 40 Adjusting mechanism; 50 Laser light source; 60 Optical component; 70 Objective lens; 80 Illumination light source; 91 Imaging device; 92 spectrophotometer; 93 Multimeter; 100 Controller

## Claims

1. A method for detecting an analyte that may be contained in a liquid sample by using a detection kit,
the detection kit including a photothermal conversion region that absorbs light and converts the light into heat,
a plurality of pores being disposed in the photothermal conversion region,
the method comprising:
introducing a plurality of microscopic objects into the plurality of pores, each of the plurality of microscopic objects having a surface modified by a host substance capable of being specifically bound to the analyte;
heating the liquid sample to generate thermal convection in the liquid sample by irradiating the photothermal conversion region with light having a wavelength within an absorption wavelength range of the photothermal conversion region; and
detecting the analyte, by monitoring the detection kit after the irradiation with the light.

2. The method for detecting an analyte according to claim 1, wherein sizes of the plurality of microscopic objects are smaller than pore diameters of the plurality of pores.

3. The method for detecting an analyte according to claim 1, wherein the plurality of microscopic objects each include a core,
the photothermal conversion region includes a thin film that converts the light into heat, and
the core has a thermal conductivity lower than a thermal conductivity of the thin film.

4. The method for detecting an analyte according to claim 1, wherein the plurality of pores are arranged in a honeycomb pattern.

5. The method for detecting an analyte according to any one of claims 1 to 4, wherein the plurality of microscopic objects each include a magnetic particle, and
the introducing includes introducing the plurality of microscopic objects into the plurality of pores by an external magnetic field.

6. The method for detecting an analyte according to any one of claims 1 to 4, wherein the introducing includes introducing the plurality of microscopic objects into the plurality of pores by irradiation with ultrasonic waves.

7. The method for detecting an analyte according to any one of claims 1 to 4, wherein the introducing includes introducing the plurality of microscopic objects into the plurality of pores by thermal convection generated by irradiating the photothermal conversion region with the light.

8. The method for detecting an analyte according to any one of claims 1 to 4, wherein the plurality of microscopic objects are larger than the liquid sample in specific gravity, and
the introducing includes introducing the plurality of microscopic objects into the plurality of pores by natural sedimentation of the plurality of microscopic objects.

9. The method for detecting an analyte according to claim 1, wherein the detecting the analyte includes:
detecting, by a receiver, light from the liquid sample after irradiation with the light; and
calculating a concentration of the analyte in the liquid sample, on a basis of a signal from the receiver.

10. The method for detecting an analyte according to claim 9, wherein the receiver includes an imaging device, and
the calculating the concentration of the analyte includes:
calculating an accumulation area of the analyte, from an image captured by the imaging device; and
calculating the concentration of the analyte, from the calculated accumulation area by referring to a correlation between the concentration of the analyte and an accumulation area of the analyte.

11. The method for detecting an analyte according to claim 10, wherein the analyte is labeled with a fluorescent dye, and
the calculating the accumulation area of the analyte includes calculating a fluorescence area.

12. The method for detecting an analyte according to claim 1, wherein the detecting the analyte includes selectively detecting the analyte, from a plurality of substances including the analyte.

13. The method for detecting an analyte according to claim 1, wherein the liquid sample is a liquid sample containing an impurity, and
the detecting the analyte includes detecting the analyte contained in the liquid sample containing the impurity.

14. The method for detecting an analyte according to claim 9, wherein the receiver includes a spectroscope that measures a spectrum of light from the liquid sample, and
calculating the concentration of the analyte includes calculating the concentration of the analyte, on a basis of the spectrum measured by the spectroscope.

15. The method for detecting an analyte according to claim 1, wherein the detection kit further includes first and second electrodes being arranged apart from each other with the photothermal conversion region interposed between the electrodes, and
the detecting the analyte includes detecting the analyte, on a basis of a change of an electric resistance between the first electrode and the second electrode.

16. A detection kit for an analyte that may be contained in a liquid sample comprising:
a photothermal conversion region that absorbs light and converts the light into heat, a plurality of pores being disposed in the photothermal conversion region; and
a plurality of microscopic objects disposed in the plurality of pores, each of the plurality of microscopic objects having a surface modified by a host substance capable of being specifically bound to the analyte.

17. A detection system for an analyte, comprising:
the detection kit according to claim 16;
a light source that emits light having a wavelength within an absorption wavelength range of the photothermal conversion region; and
a detector that detects the analyte, by monitoring the detection kit after irradiation with the light from the light source.

18. A method for manufacturing a detection kit used for detecting an analyte that may be contained in a liquid sample, the method comprising:
preparing the detection kit including a photothermal conversion region in which a plurality of pores are disposed; and
introducing a plurality of microscopic objects into the plurality of pores, each of the plurality of microscopic objects having a surface modified by a host substance capable of being specifically bound to the analyte.
